# EUROPEAN PATENT APPLICATION

(11) **EP 3 666 230 A1**
(43) Date of publication of application: **17.06.2020**
(21) Application number: 20151452.8
(22) Date of filing: 28.02.2014
(51) Int. Cl.: A61F 2/38, A61F 2/30

(54) **FIXED-BEARING KNEE PROSTHESIS HAVING INTERCHANGEABLE COMPONENTS**

(30) Priority: 07.03.2013 US 201313788921
(62) Divisional of application: 14157148.9
(71) Applicant: DePuy Ireland Unlimited Company, County Cork (IE)
(72) Inventor: DEFFENBAUGH, Daren L, Warsaw, IN 46581 (US); HAZEBROUCK, Stephen A, Warsaw, IN 46581 (US); HELDRETH, Mark A, Warsaw, IN 46581 (US); WYSS, Joseph G, Warsaw, IN 46581 (US)
(74) Representative: Pawlyn, Anthony Neil

(57) **Abstract**

A fixed-bearing knee prosthesis system includes a femoral component having a medial condyle surface and a lateral condyle surface. The system also includes a tibial tray having a platform with a fixation member extending downwardly from a lower surface thereof, the platform having (i) a generally Y-shaped posterior buttress extending upwardly from an upper surface of the platform and having a pair of arms extending along a posterior section of a perimeter of the platform, each of the pair of arms having an undercut defined therein, and (ii) an anterior buttress extending along an anterior section of the perimeter of the platform and extending upwardly from the upper surface of the platform, the anterior buttress being discontiguous with the posterior buttress and having an undercut defined therein, and (iii) an elongated bore having a superior end that opens into an upper surface of the posterior buttress. The system also includes a plurality of revision bearings configured to be separately secured to the tibial tray, wherein each of the plurality of revision bearings has (i) a width that is different from at least some of the other of the plurality of revision bearings, (ii) an upper surface having a medial bearing surface configured to articulate with the medial condyle surface of the femoral component and a lateral bearing surface configured to articulate with the lateral condyle surface of the femoral component, (iii) a lower surface having a posterior recess defined therein to receive the posterior buttress and an anterior recess defined therein to receive the anterior buttress, (iv) a reinforcing pin extending out of the posterior recess, (v) a pair of posterior locking tabs arranged to be respectively received in the undercuts defined in each of the pair of arms of the posterior buttress, and (vi) an anterior locking tab arranged to be received in the undercut defined in the anterior buttress. Also included in the system is a plurality of primary bearings configured to be separately secured to the tibial tray, wherein each of the plurality of primary bearings has (i) a width that is different from at least some of the other of the plurality of primary bearings, (ii) an upper surface having a medial bearing surface configured to articulate with the medial condyle surface of the femoral component and a lateral bearing surface configured to articulate with the lateral condyle surface of the femoral component, (iii) a lower surface having a posterior recess defined therein to receive the posterior buttress and an anterior recess defined therein to receive the anterior buttress, the lower surface being devoid of a reinforcing pin, (iv) a pair of posterior locking tabs arranged to be respectively received in the undercuts defined in each of the pair of arms of the posterior buttress, and (v) an anterior locking tab arranged to be received in the undercut defined in the anterior buttress.

## Description

The present invention relates generally to an implantable orthopaedic prosthesis, and more particularly to an implantable knee prosthesis.

During the lifetime of a patient, it may be necessary to perform a joint replacement procedure on the patient as a result of, for example, disease or trauma. The joint replacement procedure may involve the use of a prosthesis which is implanted into one or more of the patient's bones. In the case of a knee replacement procedure, a tibial tray is implanted into the patient's tibia. A bearing is secured to the tibial tray. The condyle surfaces of a replacement femoral component bear against the tibial bearing.

One type of knee prosthesis is a fixed-bearing knee prosthesis. In such a prosthesis, the bearing does not move relative to the tibial tray. Fixed-bearing designs are commonly used when the condition of the patient's soft tissue (i.e., knee ligaments) does not allow for the use of a knee prosthesis having a mobile bearing.

The components of a fixed-bearing knee prosthesis are typically provided by the manufacturer in matching sizes. Specifically, a particular size of femoral component in most currently available fixed-bearing knee prostheses can be used with different sized bearings, but each bearing size is generally matched to a particular size of tibial tray.

The invention provides a fixed-bearing knee prosthesis which includes a femoral component having a medial condyle surface and a lateral condyle surface. The prosthesis also includes a bearing having a medial bearing surface configured to articulate with the medial condyle surface of the femoral component, and a lateral bearing surface configured to articulate with the lateral condyle surface of the femoral component. A tibial tray is secured to the bearing. The tibial tray has a platform with an elongated stem extending downwardly from a lower surface thereof. The platform has a posterior buttress extending along a posterior section of a perimeter of the platform and extending upwardly from an upper surface of the platform. An anterior buttress extends along an anterior section of the perimeter of the platform and upwardly from the upper surface of the platform. The tibial tray also has an elongated bore that opens into the upper surface of the posterior buttress.

The posterior buttress is generally Y-shaped and has a first arm extending along a posterior edge of the platform and having a first undercut defined therein. A second arm extends along the posterior edge of the platform in a direction away from the first arm and has a second undercut defined therein. A third arm extends anteriorly away from the first arm and the second arm.

A first imaginary line extends along a lateral-most edge of the first arm, with a second imaginary line extending along a medial-most edge of the second arm and intersecting the first imaginary line to define an angle of intersection.

Optionally, the angle of intersection of the imaginary lines is from 45 to 145°, or between 45 and 145°. Optionally, the angle of intersection of the imaginary lines is from 60 to 120°, or between 60 and 120°. Optionally, the angle of intersection of the imaginary lines is approximately 90°.

Optionally, the anterior buttress is generally T-shaped. In such cases, the anterior buttress includes a first arm extending along an anterior edge of the platform with a first undercut defined therein, and a second arm extending along the anterior edge of the platform in a direction away from the first arm and with a second undercut defined therein. A third arm extends posteriorly away from the first arm and the second arm.

Optionally, the third arm of the posterior buttress may be contiguous with the third arm of the anterior buttress.

Optionally, the bearing may include a first posterior tab positioned in the first undercut defined in the first arm of the posterior buttress, a second posterior tab positioned in the second undercut defined in the second arm of the posterior buttress, a first anterior tab positioned in the first undercut defined in the first arm of the anterior buttress, and a second anterior tab positioned in the second undercut defined in the second arm of the anterior buttress.

Optionally, both the medial bearing surface and the lateral bearing surface are defined in the upper surface of the bearing. The lower surface of the bearing contacts the upper surface of the platform and includes a posterior recess and an anterior recess formed therein. The posterior buttress is positioned in the posterior recess and the anterior buttress is positioned in the anterior recess. The posterior recess may be contiguous with the anterior recess.

The invention also provides a fixed-bearing knee prosthesis which includes a femoral component having a medial condyle surface and a lateral condyle surface. The prosthesis also includes a bearing having a medial bearing surface configured to articulate with the medial condyle surface of the femoral component, and a lateral bearing surface configured to articulate with the lateral condyle surface of the femoral component. The bearing also has a reinforcement pin. A tibial tray is secured to the bearing. The tibial tray has a platform with an elongated stem extending downwardly from a lower surface thereof. The platform has a posterior buttress extending along a posterior section of a perimeter of the platform and extending upwardly from an upper surface of the platform. An anterior buttress extends along an anterior section of the perimeter of the platform and upwardly from the upper surface of the platform. The tibial tray also has an elongated bore that opens into the upper surface of the posterior buttress.

The posterior buttress is generally Y-shaped and has a first arm extending along a posterior edge of the platform with a first undercut defined therein, and a second arm extending along the posterior edge of the platform in a direction away from the first arm with a second undercut defined therein. A third arm extends anteriorly away from the first arm and the second arm.

The anterior buttress includes a first arm extending along an anterior edge of the platform with a first undercut defined therein. A second arm of the anterior buttress extends along the anterior edge of the platform in a direction away from the first arm of the anterior buttress with an undercut defined therein.

Optionally, the superior end of the elongated bore opens into the third arm of the posterior buttress such that the reinforcing pin extends through the third arm.

Optionally, the fixation member of the tibial tray comprises an elongated stem extending downwardly from the lower surface of the platform, and the elongated bore extends into the stem such that the reinforcing pin is positioned in the stem.

Optionally, the bearing includes a first flexible posterior tab positioned in the first undercut defined in the first arm of the posterior buttress, a second flexible posterior tab positioned in the second undercut defined in the second arm of the posterior buttress, and a flexible anterior tab positioned in the undercut defined in the anterior buttress.

Optionally, the bearing includes a posterior-stabilizing spine extending from an upper surface of the bearing, both the medial bearing surface and the lateral bearing surface are defined in the upper surface of the bearing, and the posterior-stabilizing spine is positioned between the medial bearing surface and the lateral bearing surface.

Optionally, the bearing has an upper surface and a lower surface, both the medial bearing surface and the lateral bearing surface are defined in the upper surface of the bearing, the lower surface of the bearing contacts the upper surface of the platform, the lower surface of the platform has a posterior recess and an anterior recess formed therein, the reinforcing pin extends out of the posterior recess, and the posterior buttress is positioned in the posterior recess and the anterior buttress is positioned in the anterior recess.

Optionally, the posterior buttress is discontiguous with the anterior buttress.

Optionally, a first imaginary line extends along a posterior-most edge of the first arm of the anterior buttress and a posterior-most edge of the second arm of the anterior buttress. A second imaginary line extends along the longitudinal axis of the third arm of the posterior buttress and intersects the first imaginary line to define an angle of intersection. The angle of intersection is approximately 90°.

Optionally, the anterior buttress may include a third arm extending posteriorly away from the first arm and the second arm in a direction parallel to the second imaginary line. The third arm of the posterior buttress may be contiguous with the third arm of the anterior buttress.

Optionally, the bearing may include a first posterior tab positioned in the first undercut defined in the first arm of the posterior buttress, a second posterior tab positioned in the second undercut defined in the second arm of the posterior buttress, a first anterior tab positioned in the first undercut defined in the first arm of the anterior buttress, and a second anterior tab positioned in the second undercut defined in the second arm of the anterior buttress.

Optionally, both the medial bearing surface and the lateral bearing surface are defined in the upper surface of the bearing. The lower surface of the bearing contacts the upper surface of the platform and includes a posterior recess and an anterior recess formed therein. The posterior buttress is positioned in the posterior recess and the anterior buttress is positioned in the anterior recess. The posterior recess may be contiguous with the anterior recess.

The invention also provides a fixed-bearing knee prosthesis which includes a femoral component having a medial condyle surface and a lateral condyle surface, and a tibial tray having a platform with an elongated stem extending downwardly from a lower surface thereof. The platform has a generally Y-shaped posterior buttress extending upwardly from an upper surface of the platform. A pair of arms of the posterior buttress extend along a posterior section of a perimeter of the platform. Each of the pair of arms has an undercut defined therein. The platform also has an anterior buttress extending along an anterior section of the perimeter of the platform and extending upwardly from the upper surface of the platform. The knee prosthesis also includes a plurality of bearings configured to be secured to the tibial tray. Each of the plurality of bearings has a width that is different from at least some of the other of the plurality of bearings. Each of the plurality of bearings also has an upper surface having a medial bearing surface configured to articulate with the medial condyle surface of the femoral component and a lateral bearing surface configured to articulate with the lateral condyle surface of the femoral component. A lower surface of each of the plurality of bearings has at least one recess defined therein to receive the posterior buttress and the anterior buttress. Each of the plurality of bearings also includes a pair of posterior tabs arranged to be respectively received in the undercuts defined in the pair of arms of the posterior buttress. The tibial tray also has an elongated bore that opens into the upper surface of the posterior buttress.

The recess of the bearing may define a single, contiguous recess that receives both the posterior buttress and the anterior buttress.

The anterior buttress may be generally T-shaped with a pair of arms extending along an anterior edge of the platform. Each of the pair of arms of the anterior buttress may have an undercut defined therein. Each of the plurality of bearings may further include a pair of anterior tabs arranged to be respectively received in the undercuts defined in the pair of arms of the anterior buttress.

The posterior buttress may be contiguous with the anterior buttress.

The invention also provides a fixed-bearing knee prosthesis, comprising a femoral component having a medial condyle surface and a lateral condyle surface. The prosthesis includes a tibial tray having a platform with a fixation member extending downwardly from a lower surface thereof, the platform having (i) a generally Y-shaped posterior buttress extending upwardly from an upper surface of the platform and having a pair of arms extending along a posterior section of a perimeter of the platform, each of the pair of arms having an undercut defined therein, and (ii) an anterior buttress extending along an anterior section of the perimeter of the platform and extending upwardly from the upper surface of the platform, the anterior buttress being discontiguous with the posterior buttress and having an undercut defined therein, and (iii) an elongated bore having a superior end that opens into an upper surface of the posterior buttress. The prosthesis includes a plurality of revision bearings configured to be separately secured to the tibial tray, in which each of the plurality of revision bearings has (i) a width that is different from at least some of the other of the plurality of revision bearings, (ii) an upper surface having a medial bearing surface configured to articulate with the medial condyle surface of the femoral component and a lateral bearing surface configured to articulate with the lateral condyle surface of the femoral component, (iii) a lower surface having a posterior recess defined therein to receive the posterior buttress and an anterior recess defined therein to receive the anterior buttress, (iv) a reinforcing pin extending out of the posterior recess, (v) a pair of posterior locking tabs arranged to be respectively received in the undercuts defined in each of the pair of arms of the posterior buttress, and (vi) an anterior locking tab arranged to be received in the undercut defined in the anterior buttress. The prosthesis includes a plurality of primary bearings configured to be separately secured to the tibial tray, in which each of the plurality of primary bearings has (i) a width that is different from at least some of the other of the plurality of primary bearings, (ii) an upper surface having a medial bearing surface configured to articulate with the medial condyle surface of the femoral component and a lateral bearing surface configured to articulate with the lateral condyle surface of the femoral component, (iii) a lower surface having a posterior recess defined therein to receive the posterior buttress and an anterior recess defined therein to receive the anterior buttress, the lower surface being devoid of a reinforcing pin, (iv) a pair of posterior locking tabs arranged to be respectively received in the undercuts defined in each of the pair of arms of the posterior buttress, and (v) an anterior locking tab arranged to be received in the undercut defined in the anterior buttress.

Optionally, the fixation member of the tibial tray comprises an elongated stem extending downwardly from the lower surface of the platform, and the elongated bore extends into the stem such that the reinforcing pin is positioned in the stem when one of the plurality of revision bearings is secured to the tibial tray.

Optionally, the anterior locking tab and the pair of posterior locking tabs of each of the plurality of revision bearings comprise a flexible, deflectable tab.

The invention is described below by way of example with reference to the accompanying drawings, in which:
FIG. 1 is an exploded perspective view of a fixed-bearing knee prosthesis.
FIG. 2 is a bottom perspective view of the bearing of the prosthesis of FIG. 1.
FIG. 3 is a perspective view of the tibial tray of the prosthesis of FIG. 1.
FIG. 4 is a plan view of the tibial tray of the prosthesis of FIG. 1.
FIG. 5 is a diagrammatic plan view of a number of differently sized tibial trays of the prosthesis of FIG. 1.
FIGS. 6 to 9 are similar to FIG. 4, showing other tibial trays.
FIGS. 10 to 14 are similar to FIGS. 1 to 5 respectively, showing another fixed-bearing knee prosthesis.
FIGS. 15 and 16 are bottom perspective views of a revision bearing.
FIG. 17 is a side view showing the angled installation of pin-less primary bearing to the tibial tray.
FIG. 18 is a side view showing the vertical installation of a revision bearing configured with a reinforcement pin to the tibial tray.
FIG. 19 is an exploded perspective view showing that either a pin-less primary bearing or a revision bearing configured with a reinforcement pin may be secured to the tibial tray.

Terms representing anatomical references, such as anterior, posterior, medial, lateral, superior and inferior, may be used throughout this document to refer to both the orthopaedic implants described herein and a patient's natural anatomy. Such terms have well-understood meanings in both the study of anatomy and the field of orthopaedics. Use of such anatomical reference terms in this document is intended to be consistent with their well-understood meanings unless noted otherwise.

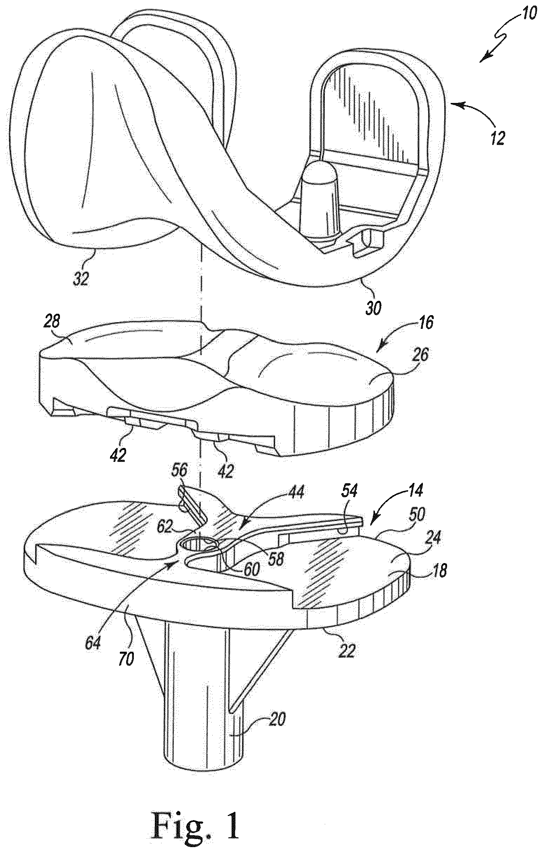

Referring to the drawings, FIGS. 1 to 4 show a fixed-bearing knee prosthesis 10. The knee prosthesis 10 includes a femoral component 12, a tibial tray 14, and a bearing 16. The tibial tray 14 includes a platform 18 having a fixation member, such as an elongated stem 20, extending away from its lower surface 22. The elongated tibial stem 20 is configured to be implanted into a surgically prepared end of a patient's tibia (not shown). Other fixation members, such as one or more short pegs or posts, may be used instead of the elongated stem 20. The bearing 16 is securable to the tibial tray 14. In particular, as will be discussed below in greater detail, the bearing 16 may be snap-fit to the tibial tray 14. In such a way, the bearing 16 is fixed relative to the tibial tray 14 (i.e., it is not rotatable or moveable in the anterior/posterior or medial/lateral directions).

The bearing 16 includes a lateral bearing surface 26 and a medial bearing surface 28. The bearing surfaces 26, 28 are configured to articulate with a lateral condyle surface 30 and a medial condyle surface 32, respectively, of the femoral component 12. Specifically, the femoral component 12 is configured to be implanted into a surgically prepared end of the patient's femur (not shown), and is configured to emulate the configuration of the patient's natural femoral condyles. As such, the lateral condyle surface 30 and the medial condyle surface 32 are configured (e.g., curved) in a manner which mimics the condyles of the natural femur. The lateral condyle surface 30 and the medial condyle surface 32 are spaced apart from one another thereby defining an intercondylar notch between them.

The components of the knee prosthesis 10 that engage the natural bone, such as the femoral component 12 and the tibial tray 14, may be constructed with a biocompatible metal, such as a cobalt chromium alloy, although other materials may also be used. The bone engaging surfaces of these components may be textured to facilitate cementing the component to the bone. Such surfaces may also be porous coated to promote bone ingrowth for permanent fixation.

The bearing 16 may be constructed with a material that allows for smooth articulation between the bearing 16 and the femoral component 12, such as a polymeric material. One such polymeric material is polyethylene such as ultrahigh molecular weight polyethylene (UHMWPE).

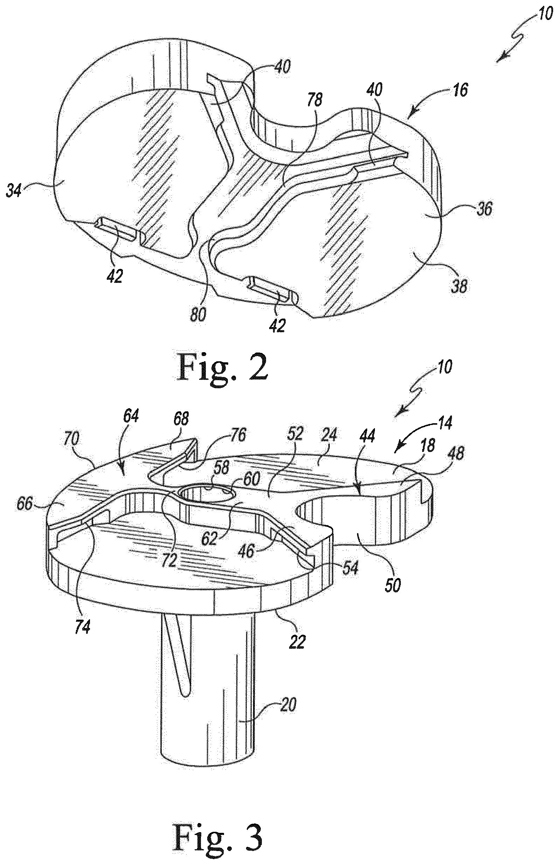

As shown in FIG. 2, the lower surface 36 of the bearing 16 includes a lateral pedestal 34 and a medial pedestal 38. The pedestals 34, 38 have a number of posterior tabs 40 defined therein. A number of anterior tabs 42 are also defined in the bearing 16.

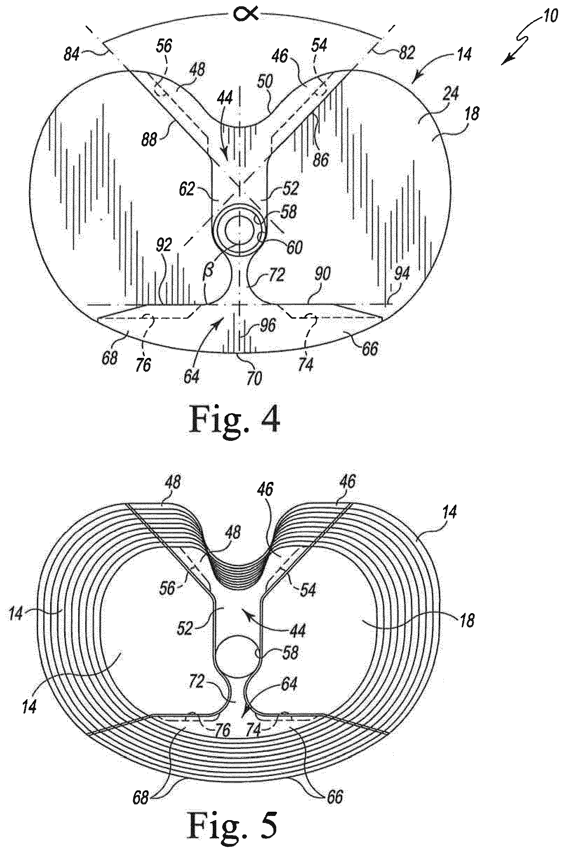

As shown in FIGS. 3 and 4, a generally Y-shaped posterior buttress 44 extends upwardly from the upper surface 24 of the tibial tray 14. In the device shown in the drawings, the posterior buttress 44 has a pair of arms 46, 48 extending along a posterior section of the perimeter of tibial tray's platform 18. Specifically, the lateral arm 46 of the posterior buttress 44 extends along the posterior edge 50 on the lateral side of the platform 18, whereas the medial arm 48 of the posterior buttress 44 extends along the posterior edge 50 on the medial side of the platform 18 in a direction away from the lateral arm 46. A third arm 52 of the posterior buttress 44 extends anteriorly away from the intersection of the lateral arm 46 and the medial arm 48 (i.e., in a direction toward the centre of the platform 18).

The posterior buttress 44 has a pair of undercuts 54, 56 defined therein. Specifically, the lateral undercut 54 is defined in the lateral arm 46 of the posterior buttress 44, with the medial undercut 56 being defined in the medial arm 48 of the posterior buttress 44.

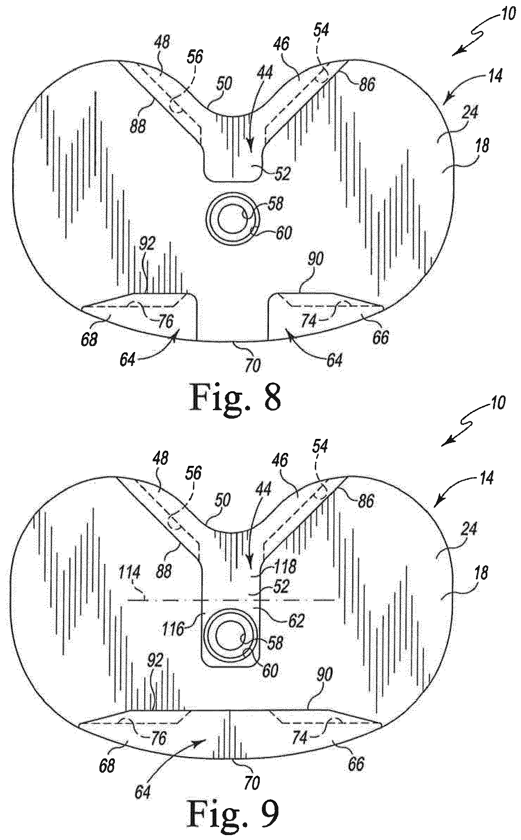

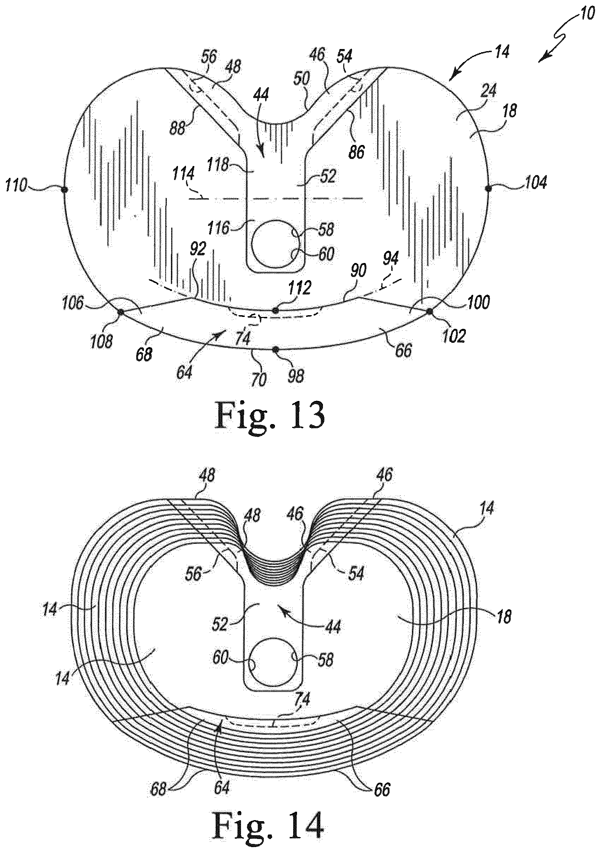

As can be seen in FIGS. 1, 3 to 10, and 12 to 14, the tibial tray 14 has an elongated bore 58 formed therein. A superior end 60 of the elongated bore 58 opens into the upper surface 62 of the posterior buttress 44. In the devices shown in FIGS. 9, 10, and 12 to 14, the superior end 60 of the elongated bore 58 opens into the third arm 52 of the posterior buttress 44. More particularly, in such devices, the superior end 60 of the elongated bore 58 opens into the anterior end of the third arm 52 of the posterior buttress 44. This is shown geometrically in FIGS. 9 and 13 in which an imaginary line 114 extending in the medial/lateral direction bisects the third arm 52 into an anterior half 116 and a posterior half 118. The superior end 60 of the elongated bore 58 opens into the anterior end of the third arm 52 of the posterior buttress 44 at a location in its anterior half 116.

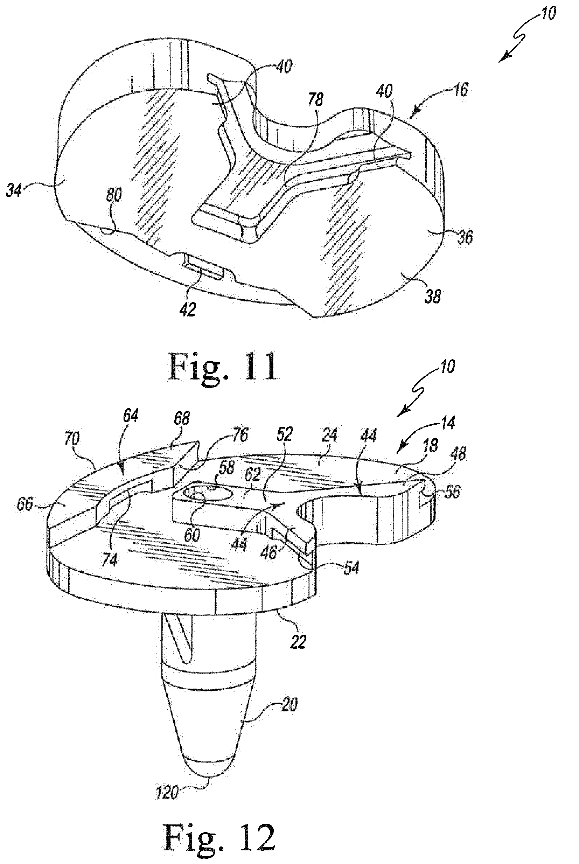

As can be seen best in FIG. 12, the elongated bore 58 extends in the superior/ inferior direction. As a result, the elongated bore 58 extends away from its superior end 60 in the direction toward the distal end 120 of the tibial tray's elongated stem 20.

As also shown in FIGS. 3 and 4, a generally T-shaped anterior buttress 64 extends upwardly from the upper surface 24 of the tibial tray 14. In the device shown in the drawings, the anterior buttress 64 has a pair of arms 66, 68 extending along an anterior section of the perimeter of tibial tray's platform 18. Specifically, the lateral arm 66 of the anterior buttress 64 extends along the anterior edge 70 on the lateral side of the platform 18, whereas the medial arm 68 of the anterior buttress 64 extends along the anterior edge 70 on the medial side of the platform 18 in a direction away from the lateral arm 66. A third arm 72 of the anterior buttress 64 extends posteriorly away from the intersection of the lateral arm 66 and the medial arm 68 (i.e., in a direction toward the centre of the platform 18).

The anterior buttress 64 has a pair of undercuts 74, 76 defined therein. Specifically, the lateral undercut 74 is defined in the lateral arm 66 of the anterior buttress 64, with the medial undercut 76 being defined in the medial arm 68 of the anterior buttress 64.

In the device shown in FIGS. 1 to 4, the posterior buttress 44 of the tibial tray 14 is contiguous with the tray's anterior buttress 64. Specifically, as shown in FIG. 4, the third arm 52 of the posterior buttress 44 is contiguous with the third arm 72 of the anterior buttress 64. However, as will be discussed below in greater detail, other arrangements are contemplated, including arrangements in which the buttresses are not contiguous. Moreover, the two buttresses 44, 64 could have similar heights or dissimilar heights.

To secure the tibial bearing 16 to the tibial tray 14, the posterior tabs 40 of the bearing 16 are positioned in the posterior undercuts 54, 56 of the tibial tray 14. The anterior portion of the tibial bearing 16 is then advanced downwardly toward the tibial tray 14 such that the anterior tabs 42 of the tibial bearing 16 are deflected by the anterior buttress 64 and then snapped into the anterior undercuts 74, 76 of the anterior buttress, so as to secure the bearing 16 to the tray 14.

As the anterior portion of the bearing 16 is advanced downwardly in such a manner, the buttresses 44, 64 of the tibial tray 14 are captured between the pedestals 34, 38 of the bearing's lower surface 36. Specifically, the lower surface 36 of the bearing 16 has a posterior recess 78 and an anterior recess 80 defined therein. The posterior recess 78 is configured to complement the shape of the posterior buttress 44 of the tibial tray 14. That is, when the bearing 16 is secured to the tibial tray 14, the sidewalls of the pedestals 34, 38 which define the posterior recess 78 contact the edges of the posterior buttress 44. Likewise, the anterior recess 80 is configured to complement the shape of the anterior buttress 64 of the tibial tray 14. The sidewalls of the pedestals 34, 38 which define the anterior recess 80 therefore contact the edges of the anterior buttress 64 when the bearing 16 is secured to the tibial tray 14. The dimensions of the recesses 78, 80 and the buttresses 44, 64 are selected such that a relatively tight fit is achieved. In such a way, the bearing 16 is fixed relative to the tibial tray 14. In particular, the configuration of the buttresses 44, 46 and the pedestals 34, 38 formed in the lower surface 36 of the bearing 16 prevent movement of the bearing 16 relative the tibial tray 14 in the anterior/posterior direction and the medial/lateral direction. Moreover, the posterior tabs positioned in the undercuts 54, 56 and the anterior tabs 42 positioned in the undercuts 74, 76 prevent lift off of the bearing 16 from the tibial tray 14. Rotational micromotion is reduced, if not prevented all together, by the relatively tight fit of the buttresses 44, 64 of the tibial tray 14 into the recesses 78, 80 of the bearing 16, particularly along the third arm 52 of the posterior buttress 44 and/or the third arm 72 of the anterior buttress 64.

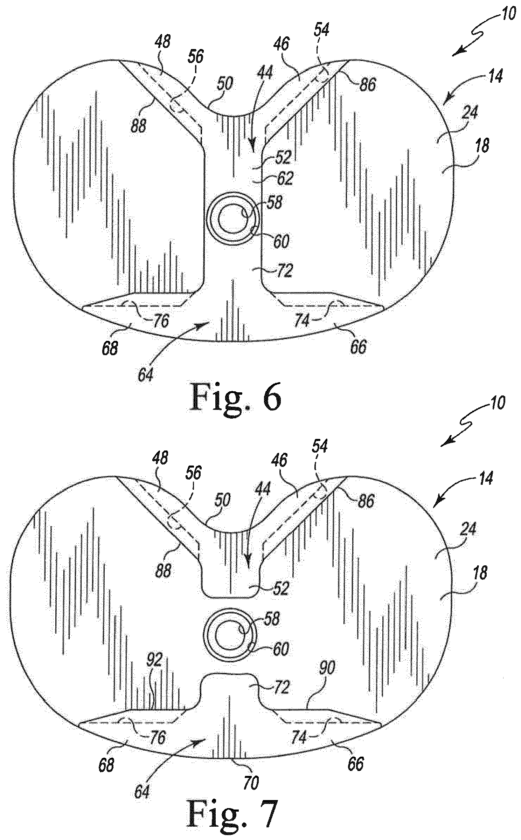

As alluded to above, in the device shown in the drawings, the posterior buttress 44 is a generally Y-shaped structure having a pair of arms 46, 48 extending in opposite directions along the posterior edge 50 of the tray's platform 18, with a third arm 52 extending anteriorly from the posterior edge 50 of the tibial tray 14 (i.e., in a direction toward the centre of the tray's platform 18). As shown in FIG. 4, the lateral arm 46 of the posterior buttress 44 includes a lateral-most edge 86, whereas the medial arm 48 of the posterior buttress 44 has a medial-most edge 88. An imaginary line 82 extends along the lateral-most edge 86 and intersects an imaginary line 84 that extends along the medial-most edge 88 to define an angle of intersection (α). In the device shown in the drawings, the angle of intersection (α) is between 45 and 145°, optionally between 60 and 120°, for example about 90°. Examples of posterior buttresses 44 are shown in FIGS. 6 to 9.

Micromotion can be reduced by increasing the angle of intersection (α) reduces micromotion. Decreasing the angle of intersection (α) increases the load bearing surface area of the tibial tray 14. It has been found that arranging the arms 46, 48 of the posterior buttress 44 as described above (i.e., having an angle of intersection (α) between 60 and 120°) provides an unexpectedly beneficial working balance between these two considerations. On particularly well-balanced arrangement of the posterior buttress 44 is found in the device shown in the drawings where the angle of intersection (α) is approximately 90°.

The anterior buttress 64 a generally T-shaped structure having a pair of arms 66, 68 extending in opposite directions along the anterior edge 70 of the tray's platform 18, with a third arm 64 extending posteriorly from the anterior edge 70 of the tibial tray 14 (i.e., in a direction toward the centre of the tray's platform 18). As shown in FIG. 4, the lateral arm 66 of the anterior buttress 64 includes a posterior-most edge 90, whereas the medial arm 68 of the anterior buttress 64 has a posterior-most edge 92. An imaginary line 94 extends along both the posterior-most edge 90 of the lateral arm 66 and the posterior-most edge 92 of the medial arm 68. An imaginary centre line 96 extends along a longitudinal axis of the third arm 52 of the posterior buttress 44. As shown in FIG. 4, the imaginary centre line 96 bisects the third arm 52 of the posterior buttress 44. The imaginary line 94 extending along the posterior-most edges 90, 92 of the arms 66, 68 of the anterior buttress 64 intersects the imaginary centre line 96 extending along the longitudinal axis of the third arm 52 of the posterior buttress 44 to define an angle of intersection (β). In the devices shown in the drawings, the arms 66, 68 (and hence the undercuts 74, 76) of the anterior buttress 64 are configured to extend in the medial/lateral direction. As a result, the angle of intersection (β) is approximately 90°. This is the case for the anterior buttresses 64 shown in FIGS. 6 to 9.

A given design of a fixed-bearing knee prosthesis is typically made commercially available in a variety of different sizes, particularly in a variety of different widths. This is done to accommodate the many variations in patient size and anatomy across a population. However, the configuration of the fixed-knee prosthesis 10 provided by the invention allows for a high degree of flexibility in relation to the sizing of the tibial tray 14 and the bearing 16. In particular, FIG. 5 is a diagrammatic representation of a plurality of differently-sized tibial trays 14 superimposed upon one another. As can be seen, despite each of the individual trays 14 having a size (e.g., width) that is different compared with the other trays 14 of the group, the basic configuration of the posterior buttress 44 and the anterior buttress 64 remains the same across the range of differently-sized trays 14. Specifically, the location of the undercuts 54, 56 defined in posterior buttress 44, respectively, remains the same across the range of differently-sized trays 14. Even though the posterior undercuts 54, 56 remain in the same location across the range of differently-sized trays 14, the width of the arms 46, 48 is varied to accommodate the overall width of a given tray 14. In a similar manner, the location of the undercuts 74, 76 defined in anterior buttress 64, respectively, remains the same across the range of differently-sized trays 14, although the width of the arms 66, 68 is varied to accommodate the overall width of a given tray 14. As shown in FIG. 5, the size and configuration of the third arms 52, 72 of the posterior buttress 44 and the anterior buttress 64, respectively, remain unchanged across the range of differently-sized trays 14.

Differently-sized bearings 16 may also be configured in such a manner. In particular, a plurality of the bearings 16 may be designed with each of such a plurality of bearings 16 having a different size, particularly a different width. However, each of such differently-sized bearings 16 may include mating features that are commonly-sized and commonly-located with the commonly-sized and commonly-located features of the tibial tray 14 described above. In particular, each of the bearings 16 across a range of differently-sized bearings 16 may include a posterior recess 78 and an anterior recess 80 that is positioned and sized to tightly fit against the edges of the posterior buttress 44 and the anterior buttress 64, respectively, of each of the tibial trays 14 across the range of differently-sized trays 14.

The posterior tabs 40 are commonly-sized and commonly-located across the range of differently-sized bearings 16 so that they are positioned in the respective posterior undercuts 54, 56 of each of the tibial trays 14 across the range of differently-sized trays 14. Likewise, the anterior tabs 42 are commonly-sized and commonly-located across the range of differently-sized bearings 16 so that they are positioned in the respective anterior undercuts 74, 76 of each of the tibial trays 14 across the range of differently-sized trays 14.

The general configuration of the buttresses 44, 64 (including contiguous variations thereof) is therefore the same across the range of differently-sized tibial trays 14. Likewise, the general configuration of the recesses 78, 80 (including contiguous variations thereof) and the general configuration of tabs 40, 42 are the same across the range of differently-sized bearings 16. Accordingly, any size bearing 16 may be secured to any size tibial tray 14. This provides the orthopaedic surgeon with greater flexibility of matching the knee prosthesis 10 to a particular patient's anatomy.

As shown in FIGS. 6 to 9, other configurations of the posterior buttress 44 and the anterior buttress 64 are also contemplated. For example, in the device shown in FIG. 6, the third arm 52 of the posterior buttress 44 and the third arm 72 of the anterior buttress 64 are configured to define a contiguous structure having a substantially constant width throughout its entire length. The recesses 78, 80 defined in the lower surface 36 of the bearing 16 are likewise reshaped in the device shown in FIG. 6 to accommodate the different shape of the buttresses 44, 64 of the tibial tray 14. In other words, while the design of the buttresses 44, 64 may be altered, it is also contemplated that the design of the recesses 78, 80 is altered accordingly to complement the configuration of the buttresses 44, 64. It is also contemplated that the general configuration of the buttresses 44, 64 shown in FIG. 6, along with the corresponding configuration of the recesses 78, 80 and tabs 40, 42 of the complementary bearing 16, may also remain the same across a range of differently-sized trays 14 and bearings 16 to accommodate the interchangeability of various sizes of trays and bearings in a similar manner to as described above with reference to FIG. 5.

As shown in FIG. 7, the third arm 52 of the posterior buttress 44 is not contiguous with the third arm 72 of the anterior buttress 64. In other words, there is a gap between the arms 52, 72. It should be appreciated that the recesses 78, 80 defined in the lower surface 36 of the bearing 16 are likewise reshaped in the device shown in FIG. 7 to accommodate the different shape of the buttresses 44, 64 of the tibial tray 14. The design of the recesses 78, 80 is altered to complement the configuration of the separated buttresses 44, 64. It is also contemplated that the general configuration of the separated buttresses 44, 64 shown in FIG. 7, along with the corresponding configuration of the recesses 78, 80 and tabs 40, 42 of the complementary bearing 16, may also remain the same across a range of differently-sized trays 14 and bearings 16 to accommodate the interchangeability of various sizes of trays and bearings in a similar manner to as described above with reference to FIG. 5.

FIG. 8 shows an anterior buttress 64 which is configured without the third arm 72. Moreover, the lateral arm 66 of the anterior buttress is spaced apart from the medial arm 68 so that there is a gap between the arms 66, 68. The recesses 78, 80 defined in the lower surface 36 of the bearing 16 are likewise reshaped in the device shown in FIG. 8 to accommodate the different shape of the buttresses 44, 64 of the tibial tray 14. In other words, the design of the recesses 78, 80 is altered to complement the configuration of the buttresses 44, 64. It is also contemplated that the general configuration of the buttresses 44, 64 of FIG. 8, along with the corresponding configuration of the recesses 78, 80 and tabs 40, 42 of the complementary bearing 16, may also remain the same across a range of differently-sized trays 14 and bearings 16 to accommodate the interchangeability of various sizes of trays and bearings in a similar manner to as described above with reference to FIG. 5.

FIG. 9 shows another knee prosthesis 10. Like the device shown in FIG. 8, the anterior buttress 64 is configured without the third arm 72. However, unlike the device of FIG. 8, the lateral arm 66 of the anterior buttress is not spaced apart from the medial arm 68, but rather is contiguous therewith. Moreover, the third arm 52 of the posterior buttress 44 is longer than that of the device shown in FIG. 8. As with the other devices described herein, the recesses 78, 80 defined in the lower surface 36 of the bearing 16 are likewise reshaped in the device of FIG. 9 to accommodate the different shape of the buttresses 44, 64 of the tibial tray 14. In other words, the design of the recesses 78, 80 is altered to complement the configuration of the buttresses 44, 64. It is also contemplated that the general configuration of the buttresses 44, 64 of FIG. 9, along with the corresponding configuration of the recesses 78, 80 and tabs 40, 42 of the complementary bearing 16, may also remain the same across a range of differently-sized trays 14 and bearings 16 to accommodate the interchangeability of various sizes of trays and bearings in a similar manner to as described above with reference to FIG. 5.

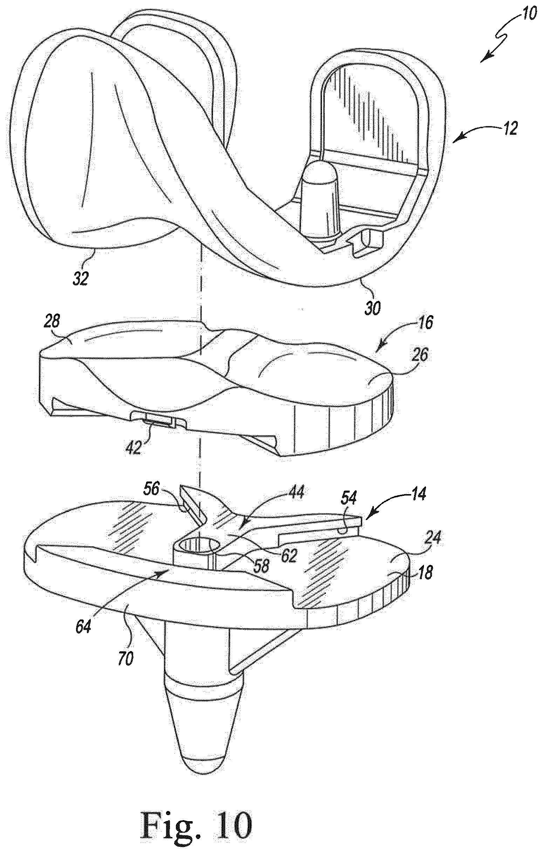

A further knee prosthesis 10 is shown in FIGS. 10 to 14. Like the devices shown in FIGS. 8 and 9, the anterior buttress 64 is configured without the third arm 72. Like the device of FIG. 9, the lateral arm 66 of the anterior buttress 64 is contiguous with the medial arm 68 of the anterior buttress 64. Specifically, as shown in FIG. 13, the anterior buttress 64 defines a continuous, monolithic structure in which proximal ends of the lateral and medial arms 66, 68 are conjoined (i.e., spatially secured to one another) at location on the anterior edge 70 at the anterior-most point 98 of the platform 18. The lateral arm 66 extends laterally away from the anterior-most point 98 of the platform and terminates at its lateral end 100 located at a point 102 on the anterior edge 70 of the platform 18 between the anterior-most point 98 of the tray's platform and the lateral-most point 104 of the platform. The medial arm 68 extends medially away from the anterior-most point 98 of the tray's platform and terminates at its medial end 106 located at a point 108 on the anterior edge 70 of the platform 18 between the anterior-most point 98 of the tray's platform and the medial-most point 110 of the tray's platform.

Unlike the device of FIG. 9, the posterior-most edge of the anterior buttress 64 of the design of FIGS. 10 to 14 is curved (i.e., arcuate-shaped). In particular, as shown most clearly in FIG. 13, the imaginary line 94 extending along the posterior-most edge 90 of the lateral arm 66 and the posterior-most edge 92 of the medial arm 68 is curved along a constant radius. It should be appreciated that since the arms 66, 68 of the anterior buttress 64 are contiguous, the posterior-most edge 90 of the lateral arm 66 and the posterior-most edge 92 of the medial arm 68 define a single, continuous, uninterrupted edge.

Moreover, the anterior buttress 64 of the design of the knee prosthesis 10 shown in FIGS. 10 to 14 includes a single anterior undercut 74 (i.e., the second undercut 76 has been omitted). The anterior undercut 74 is centred on the intersection of the two arms 66, 68 defining the anterior buttress 64. In other words, the imaginary line 94 extending along the posterior-most edge 90 of the lateral arm 66 and the posterior-most edge 92 of the medial arm 68 has a midpoint 112. The anterior undercut 74 is centred on the midpoint 112. It should be appreciated that the lower surface 36 of the bearing 16 includes a single anterior tab 42 sized and positioned to be received into the single anterior undercut 74 (see FIG. 11).

Like the devices shown in FIGS. 7 to 9, the anterior buttress 64 of the knee prosthesis 10 shown in FIGS. 10 to 14 is discontiguous with the posterior buttress 44. In other words, the buttresses 44, 64 are spaced apart from one another such that there is a gap between them. As with the other devices described herein, the recesses 78, 80 defined in the lower surface 36 of the bearing 16 are likewise reshaped in the devices of FIGS. 10 to 14 (compared with the devices shown in FIGS. 1 to 9) to accommodate the different shape of the buttresses 44, 64 of the tibial tray 14. In other words, as shown in FIG. 11, the design of the recesses 78, 80 is altered to complement the configuration of the buttresses 44, 64.

Moreover, as shown in FIG. 14, the general configuration of the buttresses 44, 64 of FIGS. 10 to 13, along with the corresponding configuration of the recesses 78, 80 and tabs 40, 42 of the complementary bearing 16, remain the same across a range of differently-sized trays 14 and bearings 16 to accommodate the interchangeability of various sizes of trays and bearings in a similar manner to as described above with reference to FIG. 5.

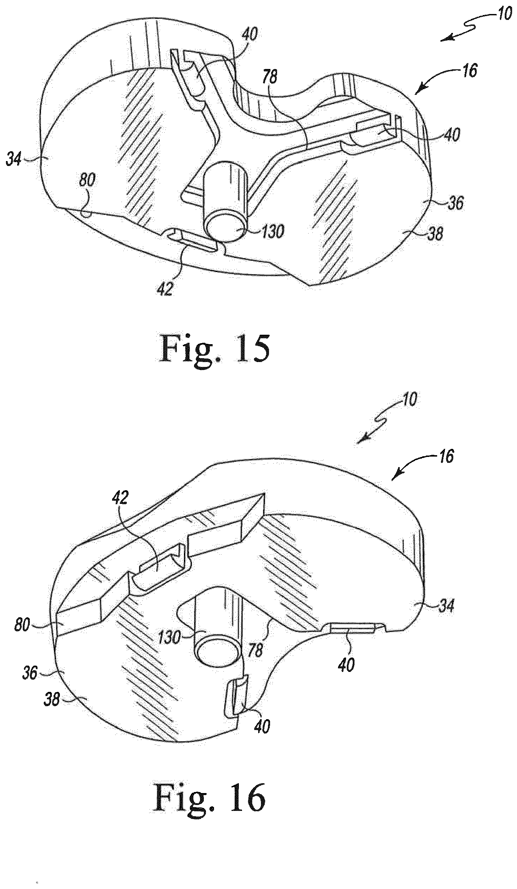

As shown in FIGS. 15 and 16, the design and interchangeability of the bearing 16 may be used in the design of a revision bearing. In the device shown in the drawings, such a revision bearing 16 may include a reinforcing pin 130 that extends downwardly from the bearing's lower surface 36. The reinforcement pin 130 may be of solid construction or may have a bore (not shown) formed therein to accommodate a stiffening pin (not shown) that may be press fit or otherwise inserted into such a bore. The stiffening pin in such an device may be constructed with a metal such as a cobalt chromium alloy.

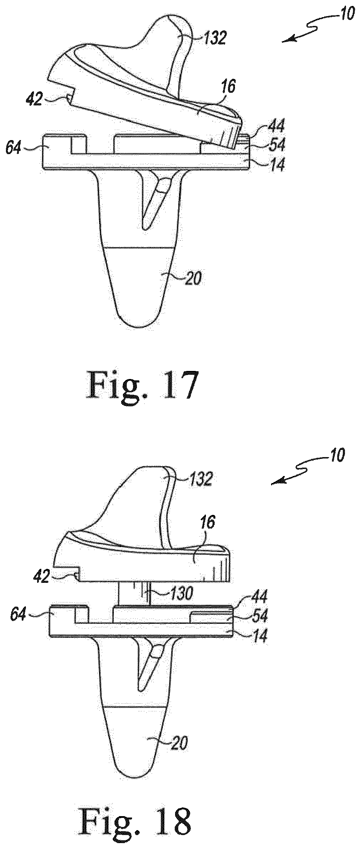

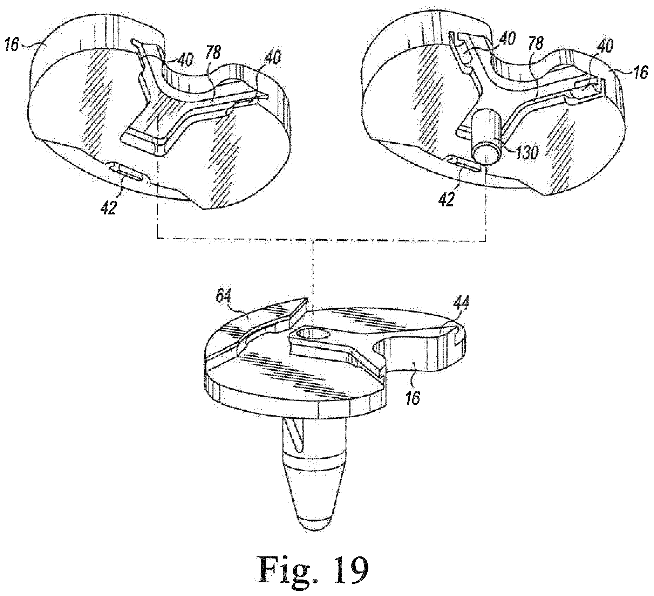

As can be seen in, for example, FIGS. 18 and 19, the reinforcing pin 130 is received into the elongated bore 58 of the tibial tray. The elongated bore 58 extends through the thickness of the platform 18 and into the stem 20. Since, as described above, the superior end 60 of the elongated bore 58 opens into the anterior end of the third arm 52 of the posterior buttress 44, the reinforcing pin 130 extends through the third arm 52 of the posterior buttress, the platform 18 and into the stem 20. In such a manner, the reinforcing pin 130 is arranged in the superior/inferior direction when the bearing 16 is secured to the tibial tray 14.

As in other devices described herein, the posterior recess 78 of the revision bearing 16 of FIGS. 15, 16, 18, and 19 is configured to complement the shape of the posterior buttress 44 of the tibial tray 14. That is, when the revision bearing 16 is secured to the tibial tray 14, the sidewalls of the pedestals 34, 38 which define the posterior recess 78 contact the edges of the posterior buttress 44. Because the superior end 60 of the tibial tray's elongated bore 58 opens into the third arm 52 of the posterior buttress 44, the reinforcing pin 130 extends out of the posterior recess 78 of the revision bearing 16 of FIGS. 15, 16, 18, and 19. Specifically, as can be seen in FIGS. 15 and 16, the reinforcing pin 130 extends out of the posterior recess 78 at a location slightly posterior to the anterior-most sidewall that defines the posterior recess 78. In such a way, the reinforcing pin 130 can be advanced into the elongated bore 58 which is similarly positioned slightly posterior to the anterior-most edge of the third arm 52 of the posterior buttress 44 of the tibial tray 14.

As can be seen in FIG. 18, the revision bearing 16 has a posterior-stabilizing spine 132 formed therein. The posterior-stabilizing spine 36 extends upwardly from the bearing's upper surface and is located between the lateral bearing surface 26 and a medial bearing surface 28. The surfaces of the posterior-stabilizing spine 132 define an anterior cam and a posterior cam that engage corresponding cam surfaces defined in the femoral component 12 to provide stability during flexion and extension of the knee prosthesis 10. As shown in FIG. 17, a primary bearing 16 may also be configured with a posterior-stabilizing spine 132. The primary bearing 16 of FIG. 17, like many of the other bearings described herein, is devoid of a reinforcing pin (i.e., it lacks a pin).

As described above and shown in FIG. 17, the pin-less primary bearings 16 described herein may be installed at an angle relative to the platform 18 of the tibial tray 14. In particular, as described above, to secure one of the pin-less primary bearings 16 to the tibial tray 14, the posterior tabs 40 of the bearing 16 are positioned in the posterior undercuts 54, 56 of the tibial tray 14. The anterior portion of the tibial bearing 16 is then advanced downwardly toward the tibial tray 14 such that the anterior tab 42 of the tibial bearing 16 is deflected by the anterior buttress 64 and thereafter snapped into the anterior undercut 74 of the anterior buttress thereby securing the bearing 16 to the tray 14. However, in the case of the revision bearing 16 configured with a reinforcing pin 130, the reinforcing pin 130 engages the tibial tray 14 first during such an angled installation thereby preventing the bearing 16 from being installed in a similar manner.

As a result, the revision bearings 16 configured with a reinforcing pin 130 are vertically installed on the tibial tray 14, as shown in FIG. 18. To accommodate such vertical installation, the posterior tabs 40 are configured in a manner similar to the flexible, deflectable anterior tabs 42, as shown in FIGS. 15 and 16. As such, to secure one of the revision bearings 16 configured with a reinforcing pin 130 to the tibial tray 14, the distal tip of the reinforcing pin 130 is aligned with, and inserted into, the superior end 60 of the tibial tray's elongated bore 58. The tibial bearing 16 is then advanced downwardly toward the tibial tray 14 such that the bearing's anterior tab 42 and posterior tabs 40 are deflected by the anterior buttress 64 and the posterior buttress 44, respectively, and then snapped into the anterior undercut 74 of the anterior buttress 64 and the posterior undercuts 54, 56 of posterior buttress 44 respectively, thereby securing the bearing 16 to the tray 14.

As in other devices described herein, the general configuration of the recesses 78, 80 and tabs 40, 42 of the revision bearings 16, remain the same across a range of differently-sized bearings 16 to accommodate the interchangeability of various sizes of trays and bearings in a similar manner to as described above with reference to FIGS. 5 and 14. Moreover, as shown in FIG. 19, such a configuration also allows for interchangeability between primary and revision bearings. In other words, both pin-less primary bearings of various sizes and various sizes of revision bearings configured with reinforcement pins can be used with various sizes of the same configuration of the tibial tray 14.

As described herein, the various designs of the knee prosthesis 10 allow for the enhanced interchangeability of differently-sized components. In particular, any one of a plurality of differently-sized bearings may be secured to any one of a plurality of differently-sized tibial trays. In some devices, any one of a plurality of differently-sized primary bearings or any one of a plurality of differently-sized revision bearings may be secured to any one of a plurality of differently-sized tibial trays. As a result, articulation surface geometries and other features of the bearing may be enhanced for each size of femoral component. Such interchangeability also allows for smaller size increments in the design of a range of femoral components.

The invention also provides a fixed-bearing knee prosthesis having features as set out in the following numbered clauses:
Clause 1: A fixed-bearing knee prosthesis, comprising:
   a femoral component having a medial condyle surface and a lateral condyle surface,
   a bearing having (i) a medial bearing surface configured to articulate with the medial condyle surface of the femoral component, (ii) a lateral bearing surface configured to articulate with the lateral condyle surface of the femoral component, and (iii) a reinforcing pin extending downwardly from a lower surface of the bearing, and
   a tibial tray secured to the bearing, the tibial tray having a platform with a fixation member extending downwardly from a lower surface thereof, the platform having (i) a posterior buttress extending along a posterior section of a perimeter of the platform and extending upwardly from an upper surface of the platform, (ii) an anterior buttress extending along an anterior section of the perimeter of the platform and extending upwardly from the upper surface of the platform, and (iii) an elongated bore having a superior end that opens into an upper surface of the posterior buttress, the reinforcing pin of the bearing being positioned in the elongated bore,
   wherein the anterior buttress has (i) a first arm extending along an anterior edge of the platform, (ii) a second arm extending along the anterior edge of the platform in a direction away from the first arm of the anterior buttress, the second arm being contiguous with the first arm, and (iii) an undercut defined therein,
   wherein the posterior buttress is generally Y-shaped and has (i) a first arm extending along a posterior edge of the platform and having a first undercut defined therein, (ii) a second arm extending along the posterior edge of the platform in a direction away from the first arm and having a second undercut defined therein, and (iii) a third arm extending anteriorly away from the first arm and the second arm, and
   wherein (i) a first imaginary line extends along a lateral-most edge of the first arm of the posterior buttress, (ii) a second imaginary line extends along a medial-most edge of the second arm of the posterior buttress and intersects the first imaginary line to define an angle of intersection therebetween, and (iii) the angle of intersection is between them.
Clause 2: The knee prosthesis of clause 1, wherein the angle of intersection is from 45-145°, optionally 60-120°, for example 90°.
Clause 3: The knee prosthesis of clause 1, wherein the superior end of the elongated bore opens into the third arm of the posterior buttress such that the reinforcing pin extends through the third arm.
Clause 4: The knee prosthesis of clause 1, wherein:
   the fixation member of the tibial tray comprises an elongated stem extending downwardly from the lower surface of the platform, and
   the elongated bore extends into the stem such that the reinforcing pin is positioned in the stem.
Clause 5: The knee prosthesis of clause 1, wherein the bearing includes:
   a first flexible posterior tab positioned in the first undercut defined in the first arm of the posterior buttress,
   a second flexible posterior tab positioned in the second undercut defined in the second arm of the posterior buttress, and
   a flexible anterior tab positioned in the undercut defined in the anterior buttress.
Clause 6: The knee prosthesis of clause 1, wherein:
   the bearing includes a posterior-stabilizing spine extending from an upper surface of the bearing,
   both the medial bearing surface and the lateral bearing surface are defined in the upper surface of the bearing, and
   the posterior-stabilizing spine is positioned between the medial bearing surface and the lateral bearing surface.
Clause 7: The knee prosthesis of clause 1, wherein:
   the bearing has an upper surface and a lower surface,
   both the medial bearing surface and the lateral bearing surface are defined in the upper surface of the bearing,
   the lower surface of the bearing contacts the upper surface of the platform,
   the lower surface of the platform has a posterior recess and an anterior recess formed therein,
   the reinforcing pin extends out of the posterior recess, and
   the posterior buttress is positioned in the posterior recess and the anterior buttress is positioned in the anterior recess.
Clause 8: The knee prosthesis of clause 7, wherein the posterior buttress is discontiguous with the anterior buttress.
Clause 9: A fixed-bearing knee prosthesis, comprising:
   a femoral component having a medial condyle surface and a lateral condyle surface,
   a bearing having (i) a medial bearing surface configured to articulate with the medial condyle surface of the femoral component, (ii) a lateral bearing surface configured to articulate with the lateral condyle surface of the femoral component, and (iii) a reinforcing pin extending downwardly from a lower surface of the bearing, and
   a tibial tray secured to the bearing, the tibial tray having a platform with a fixation member extending downwardly from a lower surface thereof, the platform having (i) a posterior buttress extending along a posterior section of a perimeter of the platform and extending upwardly from an upper surface of the platform, (ii) an anterior buttress extending along an anterior section of the perimeter of the platform and extending upwardly from the upper surface of the platform, and (iii) an elongated bore having a superior end that opens into an upper surface of the posterior buttress, the reinforcing pin of the bearing being positioned in the elongated bore,
   wherein the posterior buttress is generally Y-shaped and has (i) a first arm extending along a posterior edge of the platform and having a first undercut defined therein, (ii) a second arm extending along the posterior edge of the platform in a direction away from the first arm and having a second undercut defined therein, and (iii) a third arm extending anteriorly away from the first arm and the second arm, and
   wherein the anterior buttress has (i) a first arm extending along an anterior edge of the platform, (ii) a second arm extending along the anterior edge of the platform in a direction away from the first arm of the anterior buttress, and (iii) an undercut defined therein.
Clause 10: The knee prosthesis of clause 9, wherein the superior end of the elongated bore opens into the third arm of the posterior buttress such that the reinforcing pin extends through the third arm.
Clause 11: The knee prosthesis of clause 9, wherein:
   the fixation member of the tibial tray comprises an elongated stem extending downwardly from the lower surface of the platform, and
   the elongated bore extends into the stem such that the reinforcing pin is positioned in the stem.
Clause 12: The knee prosthesis of claim 9, wherein the bearing includes:
   a first flexible posterior tab positioned in the first undercut defined in the first arm of the posterior buttress,
   a second flexible posterior tab positioned in the second undercut defined in the second arm of the posterior buttress, and
   a flexible anterior tab positioned in the undercut defined in the anterior buttress.
Clause 13: The knee prosthesis of claim 9, wherein:
   the bearing includes a posterior-stabilizing spine extending from an upper surface of the bearing,
   both the medial bearing surface and the lateral bearing surface are defined in the upper surface of the bearing, and
   the posterior-stabilizing spine is positioned between the medial bearing surface and the lateral bearing surface.
Clause 14: The knee prosthesis of claim 9, wherein:
   the bearing has an upper surface and a lower surface,
   both the medial bearing surface and the lateral bearing surface are defined in the upper surface of the bearing,
   the lower surface of the bearing contacts the upper surface of the platform,
   the lower surface of the platform has a posterior recess and an anterior recess formed therein,
   the reinforcing pin extends out of the posterior recess, and
   the posterior buttress is positioned in the posterior recess and the anterior buttress is positioned in the anterior recess.
Clause 15: The knee prosthesis of claim 9, wherein the posterior buttress is discontiguous with the anterior buttress.

## Claims

1. A fixed-bearing knee prosthesis system, comprising:
a femoral component having a medial condyle surface and a lateral condyle surface,
a tibial tray having a platform with a fixation member extending downwardly from a lower surface thereof, the platform having (i) a generally Y-shaped posterior buttress extending upwardly from an upper surface of the platform and having a pair of arms extending along a posterior section of a perimeter of the platform, each of the pair of arms having an undercut defined therein, and (ii) an anterior buttress extending along an anterior section of the perimeter of the platform and extending upwardly from the upper surface of the platform, the anterior buttress being discontiguous with the posterior buttress and having an undercut defined therein, and (iii) an elongated bore having a superior end that opens into an upper surface of the posterior buttress,
a plurality of revision bearings configured to be separately secured to the tibial tray, wherein each of the plurality of revision bearings has (i) a width that is different from at least some of the other of the plurality of revision bearings, (ii) an upper surface having a medial bearing surface configured to articulate with the medial condyle surface of the femoral component and a lateral bearing surface configured to articulate with the lateral condyle surface of the femoral component, (iii) a lower surface having a posterior recess defined therein to receive the posterior buttress and an anterior recess defined therein to receive the anterior buttress, (iv) a reinforcing pin extending out of the posterior recess, (v) a pair of posterior locking tabs arranged to be respectively received in the undercuts defined in each of the pair of arms of the posterior buttress, and (vi) an anterior locking tab arranged to be received in the undercut defined in the anterior buttress, and
a plurality of primary bearings configured to be separately secured to the tibial tray, wherein each of the plurality of primary bearings has (i) a width that is different from at least some of the other of the plurality of primary bearings, (ii) an upper surface having a medial bearing surface configured to articulate with the medial condyle surface of the femoral component and a lateral bearing surface configured to articulate with the lateral condyle surface of the femoral component, (iii) a lower surface having a posterior recess defined therein to receive the posterior buttress and an anterior recess defined therein to receive the anterior buttress, the lower surface being devoid of a reinforcing pin, (iv) a pair of posterior locking tabs arranged to be respectively received in the undercuts defined in each of the pair of arms of the posterior buttress, and (v) an anterior locking tab arranged to be received in the undercut defined in the anterior buttress.

2. The knee prosthesis system of claim 1, wherein:
the fixation member of the tibial tray comprises an elongated stem extending downwardly from the lower surface of the platform, and
the elongated bore extends into the stem such that the reinforcing pin is positioned in the stem when one of the plurality of revision bearings is secured to the tibial tray.

3. The knee prosthesis system of claim 1, wherein the anterior locking tab and the pair of posterior locking tabs of each of the plurality of revision bearings comprise a flexible, deflectable tab.

4. The knee prosthesis system of claim 1, wherein:
each revision bearing of the plurality of revision bearings includes a posterior-stabilizing spine extending from an upper surface of the bearing,
both the medial bearing surface and the lateral bearing surface are defined in the upper surface of the bearing, and
the posterior-stabilizing spine is positioned between the medial bearing surface and the lateral bearing surface.
